# EUROPEAN PATENT SPECIFICATION

(11) **EP 1 207 206 B1**
(45) Date of publication and mention of the grant of the patent: **14.06.2006**
(21) Application number: 00949958.3
(22) Date of filing: 02.08.2000
(51) Int. Cl.: C12P 19/24

(54) **PROCESS FOR ENZYMATICALLY PRODUCING MANNOSE**
VERFAHREN ZUR ENZYMATISCHEN HERSTELLUNG VON MANNOSE
PRODUCTION DE MANNOSE PAR VOIE ENZYMATIQUE

(30) Priority: 02.08.1999 JP 25209499; 17.01.2000 JP 2000007455
(43) Date of publication of application: 22.05.2002
(73) Proprietor: Japan Science and Technology Agency, Kawaguchi-shi, Saitama 332-0012 (JP)
(72) Inventor: TAKASAKI, Yoshiyuki, Matsudo-shi, Chiba 270-0034 (JP)
(74) Representative: Calamita, Roberto
(86) International application number: PCT/JP2000/005176
(87) International publication number: WO 2001/009366

(56) References cited:
- JP-A- 4 218 370
- JP-A- 6 292 578
- JP-A- 9 191 894
- JP-B2- 63 021 678
- TAKASAKI YOSHIYUKI ET AL: "Enzymatic production of D-mannose from D-fructose by mannose isomerase" JOURNAL OF FERMENTATION AND BIOENGINEERING, vol. 76, no. 3, 1993, pages 237-239, XP002311228 ISSN: 0922-338X
- DATABASE CA [Online] CHEMICAL ABSTRACTS SERVICE, COLUMBUS, OHIO, US; 26 June 1997 (1997-06-26), SATO, TAKEHARU ET AL: "Production of D-mannose from D-fructose by Acinetobacter sp." XP002311229 retrieved from STN Database accession no. 1997:396844 & SEITO GIJUTSU KENKYU KAISHI , 44, 35-41 CODEN: SGIKA6; ISSN: 0370-9841, 1996,
- DATABASE WPI Section Ch, Week 197946 Derwent Publications Ltd., London, GB; Class D17, AN 1979-75701B XP002311230 & JP 54 129169 A (ICI AMERICAS INC) 6 October 1979 (1979-10-06)

## Description

### Technical Field

The present invention relates to a method for producing mannose from fructose in high yield by using an enzyme, namely, mannose isomerase, which interconverts D-mannose (hereinafter referred to as mannose) and D-fructose (hereinafter referred to as fructose). More particularly, the present invention relates to a method for producing mannose from fructose using mannose isomerase in the presence of a reducing sulfur-oxygen acid compound or an ammonium compound.

### Background Art

Mannose has been used as a material for mannitol synthesis and a constituent of animal cell culture medium. Further, it has been shown that mannose inhibits the growth of enterobacteria Salmonella, and its use as an additive for drinking water or feed for fowl such as chickens is being considered (R. H. Brown, in Foodstuff, June 12, vol. 10, 1989). Furthermore, since mannose improves the taste of various types of food, its application as food additive has also been considered.

Conventionally, mannose has been produced by the acid-hydrolysis of glucomannan contained in plants such as wood and devil's tongue, or by processing glucose under high temperature in the presence of a molybdate catalyst. However, such methods were problematic because the raw materials used are scarce or because the use of expensive reagents and high temperature are required, making the cost of production high. Therefore, the mannose has been extremely expensive, and its supply scarce.

The inventors of the present application have continued research in hope to establish a technique to produce mannose from fructose produced from glucose, which is in abundance and inexpensive, by using mannose isomerase, which converts mannose to fructose and vice versa, and a technique to produce mannose directly from glucose using mannose isomerase and glucose isomerase (an enzyme which converts D-glucose to fructose and vice versa).

Mannose isomerase is an enzyme, which was first found in 1956 by Palleroni, Doudoroff et al., in Pseudomonas saccharophila (J. Biol. Chem., vol. 218, p. 535, 1956). Later, the inventor of the present application and his coworkers found and reported that a bacterium identified as Xanthomonas ruburilineans also produces mannose isomerase (J. Agr. Chem. Soc. Japan, 37, 524, 1963; Agric. Biol. Chem. 28, 601, 1964). Also, the inventor has found and reported that a Streptomyces strain produces the same enzyme (Report of the Fermentation Research Institute Agency of Industrial Science and Technology, 28, 89, 1966).

Further, the inventor of the present application has invented a thermostable mannose isomerase, which is produced by a Pseudomonas bacterium, and has filed a Japanese patent application (Japanese Patent Provisional Publication No. 218370/1992).

However, in the conventional method of isomerizing fructose to mannose using mannose isomerase, the yield (isomerization rate) was as low as about 25%, causing separation and purification from the reaction solution to be difficult. Therefore, the purification method required high production cost.

The present application addresses the above-described circumstances, and its object is to solve the problems of the conventional methods and to provide a novel method for producing fructose in high yield by enhancing the isomerization rate of fructose to mannose.

### Disclosure of Invention

As the first invention to solve the above-mentioned problems, the present application provides a method for producing mannose, comprising the isomerization of fructose to mannose using a mannose isomerase in the presence of a reducing sulfur-oxygen acid compound or an ammonium compound.

Further, the present invention secondly provides the above method for producing mannose wherein fructose is isomerized to mannose using mannose isomerase in the presence of an ammonium compound selected from ammonium carbonate and ammonium bicarbonate.

Thirdly, the present invention provides the above method for producing mannose further comprising the removal of the reducing sulfur-oxygen acid compound or the ammonium compound by contacting the reaction solution to an anion-exchange resin of a sulfurous acid type or a bisulfite type, following the isomerization of fructose to mannose using mannose isomerase in the presence of a reducing sulfur-oxygen acid compound or an ammonium compound.

Furthermore, fourthly, the present invention also provides the method for producing mannose further comprising the removal of the ammonium carbonate or the ammonium bicarbonate from the reaction solution by vaporization, following the isomerization of fructose to mannose using mannose isomerase in the presence of the ammonium compound selected from ammonium carbonate or ammonium bicarbonate.

### Brief Description of Drawings

FIG. 1 shows the relationship between reaction pH and yield of mannose in Example 1 (Experiment 1) of the present application;
FIG. 2 shows the relationship between reaction temperature and yield of mannose in Example 1 (Experiment 2) of the present application;
FIG. 3 shows the relationship between the concentration of sulfite to that of the substrate (molar ratio) and the yield of mannose in Examples 3 and 4 of the present application;
FIG. 4 shows the separation of sulfite and various types of sugars from a sugar solution containing mannose and fructose, using a sulfurous acid type anion-exchange resin column in the Example of the present application;
FIG. 5 shows the separation of sulfite and various types of sugars from a sugar solution containing mannose and fructose, using a bisulfite type anion-exchange resin column in the Example of the present application;
FIG. 6 shows the effect of treatment temperature for the removal of ammonium carbonate by vaporization from the reaction solution in Example 15 of the present application.

### Best Mode for Carrying Out the Invention

The present invention is explained in more detail below by showing embodiments of the invention.

The present invention has been realized through the knowledge obtained by the intensive studies of the inventors of the present invention, that the isomerization rate of fructose to mannose is remarkably increased when the reaction is performed in the presence of a reducing sulfur-oxygen acid compound or an ammonium compound.

Specifically, the inventors of the present invention confirmed that the isomerization rate of fructose to mannose is enhanced up to 60% to 90% by performing the reaction at a pH of 5.5 to 7.0 in the presence of reducing sulfur-oxygen acid compounds such as sodium sulfite, sodium bisulfite or sodium metabisulfite (also referred to as sodium pyrosulfite).

When using, for example, ammonium carbonate or hydroxyl ammonium sulfate as the ammonium compound, it was confirmed that the isomerization rate to mannose is enhanced up to 30% to 85%, thereby allowing the production of mannose at high yield. Further, it was confirmed that, the isomerization rate to mannose is enhanced when performing the reaction in the presence of an ion-exchange resin treated with any one of the above compounds, as well, thereby allowing the production of mannose in good yield. However, no increase in yield was observed for mannose in the presence of non-reducing sulfur-oxygen acids such as sodium sulfate.

The invention of the present application has been achieved by the above-described finding.

The mannose isomerase used in the method for producing mannose of the present invention may be obtained by cultivating the above-described microorganisms of the genus Xanthomonas, Pseudomonas, Agrobacterium, Streptomyces and the like in a liquid medium containing organic nitrogen sources such as polypeptone and an organic carbon source such as glucose, at 30°C for 1 to 3 days, in accordance with methods described in, for example, Journal of Agricultural Chemical Society of Japan, Vol. 37, 524-528 (1963), Agricultural Biological Chemistry, Vol. 28, No. 9, 601-604 (1964), Journal of Fermentation and Bioengineering, Vol. 76, No. 3, 237-239 (1993), Report of the Fermentation Research Institute Agency of Industrial Science and Technology, 28, 89 (1965), Japanese Patent Provisional Publication No. 218370/1992 and the like. Mannose isomerase may be directly used by recovering the cells by centrifugation, or used after extraction from the cells by sonication or autolysis. Furthermore, after being extracted from the cell body, the mannose isomerase may be immobilized on an appropriate carrier and put into use.

In the method for producing mannose of the present invention, the yield of mannose is greatly influenced by the type of reducing sulfur-oxygen acid compound or the ammonium compound selected.

As examples of the reducing sulfur-oxygen acid compound, water-soluble salts such as sodium sulfite (Na₂SO₃), potassium sulfite (K₂SO₃), sodium metabisulfite (Na₂S₂O₅), (also known as sodium pyrosulfite or sodium disulfite), potassium metabisulfite (K₂S₂O₅) (also known as potassium pyrosulfite or potassium disulfite), sodium bisulfite (NaHSO₃), potassium bisulfite (KHSO₃), sodium hyposulfite (Na₂S₂O₄), potassium hyposulfite (K₂S₂O₄) and the like, are listed; among these, sodium sulfite, sodium bisulfite and sodium metabisulfite provide high yields of mannose, and are preferable. In terms of the isomerization rate, sodium metabisulfite (Na₂S₂O₅) is most preferable.

Further, in the method for producing mannose of the present invention, an ion-exchange resin to which ions of the above-described reducing sulfur-oxygen acid compounds are bonded may be used. The type of ion-exchange resin to which such reducing sulfur-oxygen anions are bonded is not particularly limited, but strong basic anion-exchange resins such as Amberlite IRA-400 (Rohm and Haas Company), Dowex 1-X8 (The Dow Chemical Company) and so forth, mixed resins of a strong basic anion-exchange resin and a cation-exchange resin (for example, Amberlite MB-1) and the like may be exemplified. The mixed resin is preferable, since it is effective in maintaining the pH of the reaction solution in a desirable range. When using an anion-exchange resin, it is preferable to treat the anion-exchange resin with sodium sulfite, sodium bisulfite or sodium metabisulfite, since in such cases the highest mannose yield is obtained.

The method for treating such anion-exchange resins is not particularly limited; for example, a method wherein after making the anion-exchange resin an OH type by treating with sodium hydroxide, the resultant anion-exchange resin is treated with the reducing sulfur-oxygen acid compound such as sodium sulfite, sodium bisulfite or sodium metabisulfite, followed by rinsing, is applicable. Further, the size and the like of the anion-exchange resin are not particularly limited, but the smaller the mesh size, the larger the amount of substrate per resin becomes, enabling the efficient production of mannose.

Various methods are applicable for the thus obtained reducing sulfur-oxygen acid compound-bonded anion-exchange resin to contact the reaction solution containing fructose and mannose isomerase. For example, the reaction solution may pass through a column packed with the reducing sulfur-oxygen acid compound-bonded anion-exchange resin, or the reducing sulfur-oxygen acid compound-bonded anion-exchange resin may be immersed in the reaction solution.

Further, in the method for producing mannose of the present invention, an ammonium compound may be used instead of the reducing sulfur-oxygen acid compound. Examples of such ammonium compounds are ammonium chloride, ammonium sulfate, diammonium hydrogenphosphate ((NH₄)₂HPO₄), ammonium dihydrogenphosphate (NH₄H₂PO₄), ammonium hydrogencarbonate (NH₄HCO₃), ammonium carbonate ((NH₄)₂CO₃), hydroxylammonium (also known as hydroxylamine (NH₂OH)), hydroxylammonium chloride, hydroxylammonium sulfate and the like. Furthermore, a cation-exchange resin treated with any one of these compounds may be used.

The ion-exchange resin to which an ion of the ammonium compound is bonded is not particularly limited; cation-exchange resin such as Amberlite IR-120, Amberlite IRC-150 (Rohm and Haas Company), Dowex 50-X8 (The Dow Chemical Company) and the like and mixed resin of cation-exchange resin and anion-exchange resin may be applied.

The method for treating these cation-exchange resins is not particularly limited; a method wherein after making the cation-exchange resin a Na type by treatment with sodium hydroxide or a H type by treatment with hydrochloride, the resultant cation-exchange resin is treated with the above-described ammonium salt or hydroxylammonium salt and rinsed, may be exemplified. Further, the size and the like of the anion-exchange resin are not particularly limited, but the smaller the mesh size, the larger the amount of substrate per resin becomes, enabling the efficient production of mannose.

Various methods are applicable for the thus obtained ammonium compound bonded cation-exchange resin to contact the reaction solution containing fructose and mannose isomerase. For example, the reaction solution may pass through a column packed with the ammonium compound bonded cation-exchange resin, or the ammonium compound bonded cation-exchange resin may be immersed in the reaction solution.

In the present method for producing mannose, using hydrazine or a cation-exchange resin treated with hydrazine instead of the reducing sulfur-oxygen acid compound or the ammonium compound may also enhance the yield of mannose. However, the above-described reducing sulfur-oxygen acid compound or ammonium compound is preferable since they are used as food additives and are highly safe.

The amount of these compounds to be added may vary depending on the type, the concentration of the starting substrate during reaction or the target isomerization rate to mannose. In other words, as the ratio of the compound to the starting substrate becomes larger, the isomerization rate to mannose increases. For example, when the concentration of the starting substrate is 10% or more, by adding the above-described compounds in about one to two times the molar concentration of the starting substrate, the content of mannose in the reaction solution may increased up to 40% to 90% from the conventional 25%.

In the present method for producing mannose, the timing for the addition of such compounds may be at the beginning of the reaction or at a suitable time where an appropriate amount (10% to 25%) of mannose has been produced. In the present method for producing mannose, since the inhibition reaction of the mannose isomerase reaction caused by the added compounds is not substantially large, even when the compound is added at the beginning of the reaction, mannose can be obtained at a high yield.

Further, the pH of the reaction is not particularly limited; but, when the reducing sulfur-oxygen acid compound is used, the pH affects the yield of mannose remarkably. The optimum pH for mannose isomerase is around pH 8, but because the optimum pH is considered to be related with the stability of the complex salt generated, and to be dependent on the type of the compound added, when various types of the reducing sulfur-oxygen acid compounds are used, the optimum pH may be set to an appropriate value in accordance with the concentration of the substrate or the target yield of mannose. Preferably, it is set in the range of pH 5 to pH 8.

On the other hand, when an ammonium compound is used, the pH seem to have no effect on the reaction; hence, the pH value is not particularly limited. Preferably, it is set in the range of pH 5.5 to pH 9.

In the method for producing mannose of the present invention, the reaction temperature also affects the yield of mannose. Therefore, it is preferable to perform the reaction at a temperature in the range of 40°C to 60°C, at which high yields are obtained. It goes without saying that the reaction may be performed in other temperature ranges than the above-described temperature range.

Further, in the method for producing mannose of the present invention, since the yield of mannose may decreased in the presence of oxygen when reducing sulfur-oxygen acid is used, it is preferable to perform the reaction under as inert an atmosphere as possible. For example, the reaction may preferably be performed under an atmosphere of nitrogen gas or helium gas.

In the present method for producing mannose, by removing the reducing sulfur-oxygen acid compound or the ammonium compound, a higher purity may be attained.

Heretofore, a method of separating mannose by forming a sodium bisulfite (NaHSO₃) complex of mannose has been reported (Japanese Patent Publication No. 21678/1988). This method utilizes the stable crystalline complex formed by mannose under strong acidic conditions (pH 3.5 or less) created by the sodium bisulfite; the crystals are then separated by centrifugation and bisulfite is oxidized into sulfuric acid using hydrogen peroxide to produce free mannose.

In the present method for producing mannose, mannose may be obtained at high yield without using such strong-acid conditions. Specifically, by using the weak affinity between the reducing sulfur-oxygen acid compound and mannose at around pH 6, and performing the conversion of fructose to mannose in the presence of mannose isomerase, the yield of mannose may be enhanced.

In the present method for producing mannose, the method for separating the mannose produced and the salts such as the reducing sulfur-oxygen acid compound, the ammonium compound and the like may be, for example, a method by addition of calcium chloride to precipitate the salt in the form of a calcium salt, which is then removed as an insoluble salt; a method of removal by cation-exchange resin, anion-exchange resin or electrodialysis using an ion-exchange membrane; a method by column chromatography using cation-exchange resin bonded with a metallic ion such as calcium,or anion-exchange resin of a sulfurous acid (SO₃⁻) type or a bisulfite (HSO₃⁻) type. Further, since the solubility of the salt (sulfite) of a sulfur-oxygen acid compound is low in alcohol, it is possible to separate the salt by adding alcohol to a reaction solution containing the salt of the sulfur-oxygen acid compound. Among the various types of purification methods, the method of using anion-exchange resin of the sulfurous acid type or the bisulfite type is preferable, since the separation efficiency is high.

For the method for contacting the anion-exchange resin of the sulfurous acid type or the bisulfite type and the reaction solution, one may pass the reaction solution through a column packed with the anion-exchange resin treated with the same compound added to the reaction solution, or one may immerse the above-described ion-exchange resin in the reaction solution. Further, the above-described ion-exchange resin and an immobilized mannose isomerase may be packed in a column, the pH adjusted, after which the fructose or a fructose-containing solution is passed through the column continuously. By using such a method, the conversion of fructose to mannose may be enhanced.

Further, in the method for producing mannose of the present invention, when a highly volatile ammonium carbonate or ammonium bicarbonate is used as the ammonium compound in the reaction, it is possible to remove the ammonium compound from the reaction solution by heating the reaction solution. However, since sugars such as fructose and the like tend to decompose and discolor by heating, it is preferable to remove such ammonium compounds under reduced pressure. By reducing pressure, the ammonium compound may be thoroughly removed even at as low a temperature as 40°C, in a short period of time. In such a case, the efficiency of the ammonium compound removal depends on the extent to which the pressure is reduced; however, it is not always necessary to reduce the pressure very much, and even a pressure of about 30 mmHg may be satisfactory.

In the method for producing mannose of the present invention, the sulfite salt or ammonium salt separated by the above-described methods and the remaining starting substrate, fructose, may be reused. Further, the reaction solution may be used as it is as a starting substrate for the production of mannitol.

Further, in the present method for producing mannose, as the starting substrate, not only fructose, but also a fructose-containing solution such as a mixed sugar solution of fructose and mannose (75% to 100% of fructose and 25% to 0% of mannose), a glucose isomerized sugar, an invert sugar and the like may be used.

Furthermore, in the present method for producing mannose, the reaction may be executed by using, as an enzyme, not only mannose isomerase but also a combination of mannose isomerase and glucose isomerase. In other words, as the starting substrate, glucose or a glucose-containing liquid such as an isomerized sugar, an invert sugar or the like may also be used to directly produce mannose.

Hereinafter, the present invention is described in more detail using examples; however, it goes without saying that the present invention is by no means limited to these

### examples.

### Examples

In the examples shown below, the enzyme activity is measured in accordance with the following method:

### <Method of measuring enzyme activity>

To 0.5 ml of a 0.1M phosphate buffer (pH 7.0) containing 0.2M mannose dissolved therein was added an appropriate amount of an enzyme, followed by the addition of water, making the total volume 1.0 ml, after which it was subjected to reaction at 50°C. After the reaction was terminated by adding 0.5M perchloric acid, the amount of fructose produced therein was determined by the cysteine-carbazole method. The amount of enzyme producing 1 µM of fructose in a minute under the above-described condition was defined as one unit.

### <Example 1>

200 ml of medium (pH 6) consisting of 2% of polypeptone, 1% of glucose, 0.2% of yeast extract, 0.3% of K₂HPO₄, and 0.03% of MgSO₄·7H₂O was put into an Erlenmeyer flask of 1 liter capacity, sterilized in an autoclave at 121°C for 15 minutes, inoculated with Agrobacterium tumefaciens (IFO 12664, ATCC 4718) and subjected to cultivation by shaking culture at 30°C for 3 days.

After cultivation, the resultant cells were recovered by centrifugation, and rinsed and suspended in distilled water. A part of the suspension was separated and the cells were disrupted by a 20KC ultrasonic cell disintegrator; then, the activity of the resultant mannose isomerase extracted was measured. It was determined that 1 ml of the resultant suspension solution contained 0.7 unit of the above-described enzyme.

The following experiments were conducted using this cell-suspension solution.

### Experiment 1

Using the thus-prepared mannose isomerase, the relationship of the yield of mannose and the reaction pH in the presence of sodium sulfite was examined.

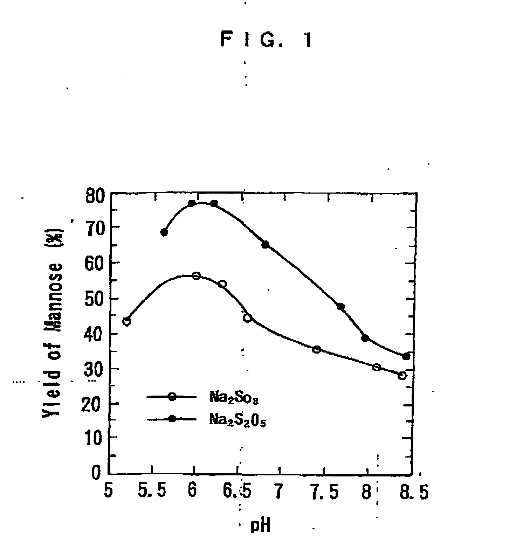

To 0.45 ml (160 mg, 0.9 M) of a 40% fructose solution was added 100 mg (0.8 M) of sodium sulfite (Na₂SO₃) and 0.1 ml (0.07 unit) of the above-described mannose isomerase; distilled water was added, making the total volume about 1 ml, after which the solution was subjected to reaction at 50°C for 20 hours. The content of the resultant mannose produced in the reaction solution was determined by high performance liquid chromatography. Results are shown in Table 1 and FIG. 1.

**Table 1**

| Reaction pH | Yield of mannose |
|---|---|
| | (%) |
| 4.6 | 26.1 |
| 5.2 | 43.6 |
| 6.0 | 56.3 |
| 6.3 | 54.1 |
| 6.6 | 44.9 |
| 6.8 | 41.5 |
| 7.4 | 36.1 |
| 8.1 | 31.2 |
| 8.4 | 28.7 |

As is apparent from Table 1 and FIG. 1, the yield of mannose was remarkably affected by the reaction pH, whereupon it was confirmed that the optimum pH at which the yield of mannose was maximum was around 6.

Further, the yield of mannose in the absence of sodium sulfite (pH 7.0, 50°C) was 25.4%.

### Experiment 2

In the same manner as in Experiment 1, the reaction of fructose isomerization in the presence of sodium sulfite using mannose isomerase was performed, and the relationship between the yield of mannose and the reaction temperature was examined.

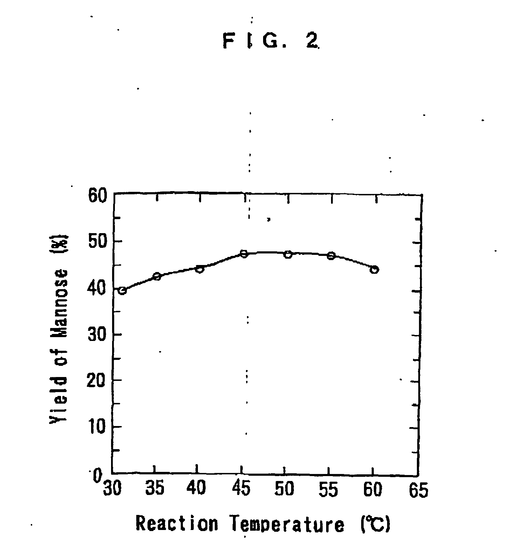

To 0.40 ml (160 mg, 0.9 M) of a 40% substrate solution used in Experiment 1 was added 100 mg (0.8 M) of sodium sulfite (Na₂SO₃) and 0.1 ml (0.07 unit) of a mannose isomerase; distilled water was added, making the total volume about 1 ml (pH about 6.0), after which the solution was subjected to reaction at various temperatures for 20 hours. The amount of the resultant mannose produced was determined by high performance liquid chromatography. Results are shown in Table 2 and FIG. 2.

**Table 2**

| Temperature (°C) | Yield of mannose |
|---|---|
| | (%) |
| 31 | 39.5 |
| 35 | 42.6 |
| 40 | 44.2 |
| 45 | 47.6 |
| 50 | 47.6 |
| 55 | 47.2 |
| 60 | 44.4 |

In terms of the activity of the mannose isomerase used in the Example, the optimum temperature was about 60°C; however, as shown in Table 2 and FIG. 2, the yield of mannose was affected by the reaction temperature and, in this reaction, the temperature range of 45°C to 55°C was optimum.

### Experiment 3

Under the same conditions as in the above-described Experiments 1 and 2, the reaction for producing mannose in the presence of sodium sulfite was performed to examine the effect of the concentration of the starting substrate (fructose).

### Table 3

| Substrate Concentration | Sodium sulfite | Yield of mannose |
|---|---|---|
| (M) | (M) | (%) |
| 0.1 | 0.1 | 38.6 |
| 0.5 | 0.5 | 42.4 |
| 1.0 | 1.0 | 46.8 |
| 1.8 | 1.8 | 49.2 |

Fructose and sodium sulfite were mixed so that the respective concentrations were as shown in Table 3; 0.2 ml (0.14 unit) of mannose isomerase was added, and the pH was adjusted to be about 6.0. Then, distilled water was added, making the total volume about 1 ml each. The solutions were subjected to isomerization reaction at 50°C for 20 hours. The content of the resultant mannose produced in the reaction solution was determined by high performance liquid chromatography. Results are shown in Table 3.

Each reaction was performed so that the starting substrate, fructose, and sodium sulfite were present in equimolar concentrations. As the concentration of the substrate became higher, the content of mannose in the reaction solution tended to increase. In other words, it was indicated that the yield of mannose per sodium sulfite increases when the reaction is performed under higher concentrations of the substrate.

### <Example 2>

In the same manner as in Example 1, the reaction for isomerization was performed using sodium metabisulfite (also known as sodium pyrosulfite or sodium disulfite Na₂S₂O₅) instead of sodium sulfite at a pH of from 5.6 to 8.2.

To 0.45 ml (180 mg) of the substrate solution used in Example 1 was added 270 mg of sodium metabisulfite and 0.1 ml (0.07 unit) of mannose isomerase. The solution was then reacted at 50°C for 45 hours. The results obtained are shown in Table 4 and FIG. 1.

**Table 4**

| Reaction pH | Yield of mannose |
|---|---|
| | (%) |
| 5.6 | 62.4 |
| 5.9 | 73.3 |
| 6.6 | 65.0 |
| 7.3 | 53.2 |
| 7.6 | 44.3 |
| 8.2 | 43.2 |

### <Example 3>

The reaction was performed in the same manner as in Examples 1 and 2 with various amounts of sodium sulfite per starting substrate (fructose).

To 0.45 ml (180 mg) of the 40% fructose solution used in Example 1 was added respective amounts of sodium sulfite (Na₂SO₃) as shown in Table, and 0.1 ml (0.07 unit) of mannose isomerase to produce respective mixtures, each with a total volume of about 1 ml; then, the mixtures were subjected to reaction at a pH of about 6.0 and at a temperature of 50°C for 20 hours.

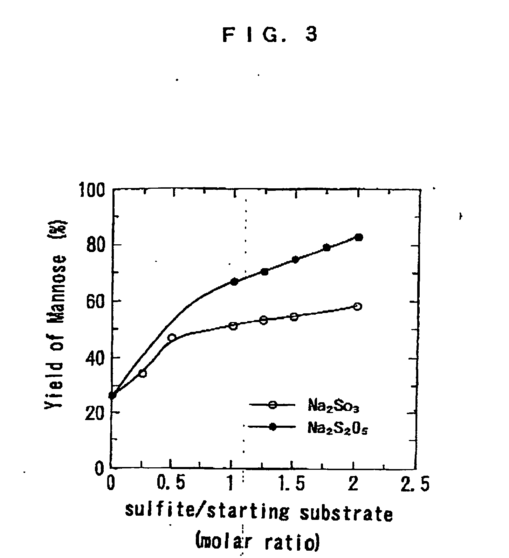

The content of the resultant mannose produced in the reaction solution was determined by high performance liquid chromatography; results are shown in Table 5 and FIG. 3.

Table 5 and FIG. 3 indicate that, as the amount of sodium sulfite per starting substrate increases, the amount of mannose increases. When sodium sulfite of 1.5 times (in molar concentration) the volume of the starting substrate was added, about 55% of fructose was isomerized into mannose; when sodium sulfite of 2.0 times the amount of the starting substrate was added, about 59% of fructose was isomerized into mannose.

**Table 5**

| Na₅SO₃/Substrate Concentration | Yield of mannose |
|---|---|
| (M/M) | (%) |
| 0 | 25.4 |
| 0.25 | 34.2 |
| 0.50 | 47.0 |
| 1.00 | 51.3 |
| 1.25 | 53.5 |
| 1.50 | 55.0 |
| 2.00 | 58.7 |

### <Example 4>

The same reaction as in Example 3 was performed using sodium metabisulfite instead of sodium sulfite.

To 0.45 ml (180 mg) of the 40% fructose solution used in Example 1 was added respective amounts of sodium metabisulfite (Na₂S₂O₅) as shown in the Table and 0.1 ml (0.07 unit) of the mannose isomerase prepared in Example 1; the pH was adjusted to be about 6.0 and the solution was subjected to reaction at 50°C for 20 hours.

The content of the resultant mannose produced in the reaction solution was determined by high performance liquid chromatography; results are shown in Table 6 and FIG. 3.

As the amount of sodium metabisulfite increased, the content of mannose in the reaction solution increased gradually; when sodium metabisulfite of 1.5 times, 1.75 times and 2.0 times the molar amount of the starting substrate were added, contents of mannose in the reaction solution were about 75%, about 80% and about 83%, respectively.

**Table 6**

| Na₃SO₃/Substrate Concentration | Yield of mannose |
|---|---|
| (M/M) | (%) |
| 0 | 25.4 |
| 1.00 | 66.9 |
| 1.25 | 70.5 |
| 1.50 | 75.4 |
| 1.75 | 79.7 |
| 2.00 | 83.1 |

The above-described results indicate that by using sodium metabisulfite fructose is isomerized into mannose at a higher isomerization rate than by using sodium sulfite.

### <Example 5>

To 0.2 ml (80 mg), 0.4 ml (160 mg) and 0.8 ml (320 mg) of the 40% fructose solution used in Example 1 was added 0.1 ml (0.07 unit) of mannose isomerase; distilled water was added, making the total volume about 1 ml each. After 1 g (wet) of Dowex 1-X8 of the sulfite type treated with sodium sulfite (Na₂SO₃) was added to each solution, the solutions were subjected to reaction at a pH of about 6.0 and at 40°C for 20 hours.

After the reaction, the content of resultant mannose produced in the reaction solution was determined by high performance liquid chromatography; results are shown in Table 7.

Increase of mannose was also observed in the reaction using an anion-exchange resin of a sulfurous acid-type.

**Table 7**

| Dowex 1-X8 | Substrate Concentration | Yield of mannose |
|---|---|---|
| (wet g) | (mg) | (%) |
| No addition | 160 | 25.0 |
| 1 | 80 | 49.5 |
| 1 | 160 | 38.0 |
| 1 | 320 | 30.2 |

### <Example 6>

Amberlite MB-1 ion-exchange resin (Rohm and Haas Company; available from Organo Corporation) which is a mixture of equal amounts of a strong acidic cation-exchange resin and a strong basic anion-exchange resin was treated with sodium metabisulfite, and packed in a column (diameter of 2.0 cm x length of 2.5 cm). To the column, 2.5 ml of the 40% fructose solution used in Example 1 and 0.5 ml (0.35 unit) of a mannose isomerase was added, and the solution was subjected to reaction at 50°C at a pH of 6.0 to 6.2.

After 5 hours of reaction, the content of the resultant mannose produced in the reaction solution was determined by high performance liquid chromatography; 38.5% of fructose was isomerized into mannose.

Further, the above-described ion-exchange resin could be reused without regeneration.

### <Example 7>

Production of mannose was performed using glucose as the starting substrate in the presence of mannose isomerase and glucose isomerase.

180 mg (1 M amount) of glucose, 189 mg (1.5 M amount) of sodium sulfite, 0.05 ml (0.005 M amount) of 0.1 M MgSO₄, 20 mg of a commercially available immobilized glucose isomerase preparation (available from Nagase Biochemical Sales Co., Ltd.) and 0.1 ml (0.07 unit) of mannose isomerase prepared in Example 1 were mixed, after which distilled water was added, making the total volume about 1 ml, and the pH adjusted to 6.0 to 6.2. The solution was subjected to reaction at 50°C for 20 hours.

Contents of the resultant mannose, fructose and glucose in the reaction solution were determined by high performance liquid chromatography; the content were 29.5%, 26.7% and 43.8%, respectively. In other words, compared to the yield of mannose (12.0%) when sodium sulfite was not added, a sugar solution containing twice or more mannose was obtained.

### <Example 8>

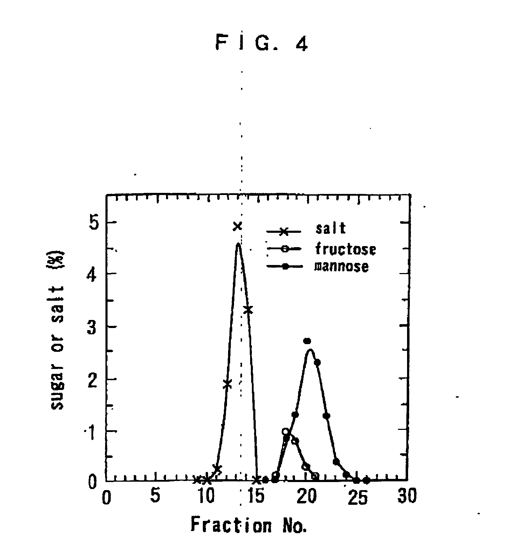

1 ml of a sugar liquid of about 1M, which was prepared in Example 4, containing about 75% of mannose and about 25% of fructose was supplied to a column (diameter of 1.5 cm X length of 25 cm) packed with a sulfurous acid-type (SO₃) Dowex 1-X8 which had previously been heated to 40°C, eluted with water (flow rate about 8 ml/hour; fraction volume about 2 ml). The results are shown in FIG. 4.

As is apparent from FIG. 4, the salt and the sugar were almost completely separated from each other.

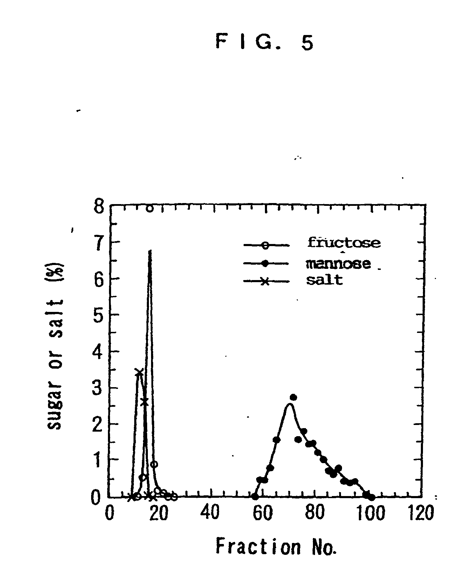

FIG. 5 is a graph showing the result when the salt and the sugar were separated (flow rate about 8 ml/hour; fraction volume about 2 ml) using a column (diameter of 1.5 cm X length of 25 cm) packed with a bisulfite-type (HSO₃) Dowex 1-X8 instead of the above-described sulfurous acid-type Dowex 1-X8.

As is apparent from FIG. 5, mannose could almost completely be separated from fructose and the salt.

It was confirmed that the separation was more complete when a column packed with the bisulfite-type (HSO₃) anion-exchange resin was used than when a column packed with a sulfurous acid-type (SO₃) anion-exchange resin was used.

Further, also from the sugar solution containing salts other than a sulfite or bisulfite, namely, a salt of sulfur-oxygen acid or an ammonium salt, each ingredient could each be isolated in the same manner.

### <Example 9>

To 0.2 ml (160 mg) of an 80% sugar solution (contents of mannose and fructose being 20% and 80%, respectively) consisting of 64% of fructose and 16% of mannose, was added 50 mg each of ammonium chloride, ammonium phosphate and ammonium carbonate and 0.15 ml (0.11 unit) of an enzyme liquid. Distilled water was added, making the total volume about 1 ml each, and the solutions were subjected to reaction at 45°C for 20 hours.

The content of the resultant mannose produced in the reaction solution was determined by high performance liquid chromatography; results are shown in Table 8.

**Table 8**

| Ammonium salt | Yield of mannose |
|---|---|
| | (%) |
| No addition | 25.5 |
| Ammonium chloride | 29.5 |
| Ammonium sulfate | 41.0 |
| Ammonium phosphate | 35.0 |
| Ammonium carbonate | 36.1 |

As is apparent from Table 8, while the yield of mannose when an ammonium compound was not added was 25.5%, the yield of mannose for the reaction wherein any one of ammonium compounds was added increased remarkably.

### <Example 10>

To 0.22 ml (180 mg; 1.0 M amount) of the substrate solution used in Example 9 was added 0, 0.25 M, 0.5 M and 1 M of ammonium sulfate, ammonium carbonate and ammonium bicarbonate as well as an enzyme solution (0.15 ml; 0.11 unit). Distilled water was aded making the total volume about 1 ml (pH being about 7.0) each. The solutions were subjected to reaction at 50°C for 20 hours. After the reaction, the resultant mannose produced was determined by high performance liquid chromatography; results are shown in Table 9.

**Table 9**

| Ammonium salt | Addition amount | Yield of mannose |
|---|---|---|
| | (M) | (%) |
| Reference (no addition) | | 25.5 |
| Ammonium sulfate | 0.25 | 27.6 |
| | 0.50 | 31.5 |
| | 1.00 | 32.1 |
| Ammonium bicarbonate | 0.25 | 28.4 |
| | 0.50 | 30.1 |
| | 1.00 | 36.7 |
| Ammonium carbonate | 0.25 | 31.2 |
| | 0.50 | 35.5 |
| | 1.00 | 42.0 |
| | 1.50 | 54.8 |
| | 2.00 | 53.4 |

As is apparent from Table 9, as the amount of any of ammonium compounds increased, the yield of mannose increased; the yield of mannose when ammonium carbonate of 1.5 times the substrate concentration was added, was about 55%.

### <Example 11>

In the present Example, the reaction using hydroxylammonium salt as the ammonium compound is described.

To 0.2 ml (160 mg) of the substrate solution used in Example 9 was added 0.5 M or 1.0 M of hydroxylammonium chloride or hydroxylammonium sulfate and 0.15 ml (0.11 unit) of the above-described mannose isomerase solution. Distilled water was added, making the total volume about 1 ml. The solutions were subjected to reaction at 45°C for 20 hours. The content of the resultant mannose produced in the reaction solution was determined by high performance liquid chromatography; results are shown in Table 10.

**Table 10**

| Hydroxylammonium salts | Addition amount | Yield of mannose |
|---|---|---|
| | (M) | (%) |
| No addition | 0.0 | 25.5 |
| Hydroxylammonium chloride | 0.5 | 47.3 |
| | 1.0 | 61.5 |
| Hydroxylammonium sulfate | 0.5 | 74.0 |
| | 1.0 | 55.3 |

As is apparent from Table 10, the yield of mannose increased remarkably by the addition of the hydroxylammonium salt; a sugar solution containing 60% to 75% of mannose was obtained.

### <Example 12>

The reaction was performed using a cation-exchange resin treated with an ammonium compound selected from ammonium chloride, hydroxylammonium and hydrazine.

Amberlite IR-120 (being about 30 mesh; available from Rohm and Haas Company) was treated with 1N hydrochloric acid, making it a H type resin, treated with ammonium chloride, hydroxylammonium or hydrazine, and rinsed with distilled water; 1 g (wet) of the thus-prepared resin was put into separate test tubes, to which 0.2 ml (160 mg) of the starting substrate solution used in Example 9 and 0.15 ml (0.11 unit) of the above-described enzyme liquid were added, making the total volume about 1 ml each. The solutions were subjected to reaction at 45°C for 20 hours. Results are shown in Table 11.

**Table 11**

| Ion-exchange resin type | Mannose content |
|---|---|
| | (%) |
| Reference (no addition) | 25.8 |
| Ammonium chloride treated | 34.8 |
| Hydroxylammonium treated | 58.0 |
| Hydrazine treated | 56.9 |

As is apparent from Table 11, by contacting the reaction solution with any one of the ammonium type, hydroxylammonium type or hydrazine type cation-exchange resin, the yield of mannose was increased remarkably.

### <Example 13>

1 g (wet) of the hydroxylammonium-treated Amberlite IR-120 used in Example 12, 0.1 ml (80 mg), 0.2 ml (160 mg), 0.3 ml (240 mg), 0.4 ml (320 mg) and 0.5 ml (400 mg) each of the starting substrate solution and 0.15 ml (0.11 unit) of the enzyme liquid were mixed, the total volume made up to about 1 ml each, and the mixture was subjected to reaction at 45°C for 20 hours. The results obtained are shown in Table 12.

**Table 12**

| Amount of Ion-exchange resin | Substrate amount | Mannose content |
|---|---|---|
| (g. wet) | (mg) | (%) |
| Reference (No addition) | | 25.8 |
| 1 | 80 | 58.5 |
| 1 | 160 | 53.1 |
| 1 | 240 | 53.8 |
| 1 | 320 | 44.3 |
| 1 | 400 | 29.0 |

From Table 12, it was confirmed that, by using appropriate amounts of the substrate per resin (about 80 mg of the substrate against 1 g of the resin in a wet state), a sugar solution with a mannose content of about 59% could be obtained.

### <Example 14>

Amberlite IRC-50 (being about 30 mesh; available from Rohm and Haas Company) treated with 1N hydrochloric acid to make it a H type, was treated with ammonium chloride or hydroxylammonium and rinsed with distilled water; 1 g (wet) of the-thus prepared resin was put into separate test tubes, to which 0.2 ml (160 mg) of the starting substrate used in Example 1 and 0.1 ml (0.11 unit) of the above-described enzyme liquid were added, making the total volume about 1 ml each; the solutions were subjected to reaction at 45°C for 20 hours. Results are shown in Table 13.

**Table 13**

| Ion-exchange resin type | Mannose content |
|---|---|
| | (%) |
| Reference (no addition) | 25.6 |
| Ammonium type | 30.7 |
| Hydroxylammonium type | 74.7 |

From Table 13, it was confirmed that, even when a weak acidic ion-exchange resin of the ammonium type or the hydroxylammonium type was used, the yield of mannose increased remarkably.

### <Example 15>

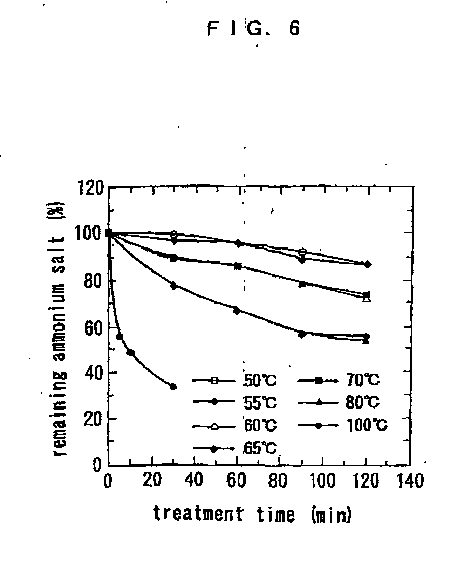

2 ml of a reaction solution containing 6.8% of mannose, 2.2% of fructose and 10.0% of ammonium carbonate was put into separate test tubes (each having a diameter of 14 mm X length of 100 mm) and heated at a temperature of 50°C, 55°C, 60°C, 65°C, 70°C, 80°C and 100°C. A predetermined volume was taken out of each of the test tubes at certain intervals of time and the amount of the remaining ammonium carbonate therein was determined by a densimeter. The results obtained are shown in Table 14 and FIG. 6.

From Table 14 and FIG. 6, it was confirmed that by heating at 60°C or more, ammonium carbonate is vaporized and removed from the product (mannose) solution.

**Table 14**

| Treatment time (min) | Amount of remaining ammonium carbonate | | | | | | |
|---|---|---|---|---|---|---|---|
| | Treatment Temperature | | | | | | |
| | 50°C | 55°C | 60°C | 65°C | 70°C | 80°C | 100°C |
| 0 | 100 | 100 | 100 | 100 | 100 | 100 | 100 |
| 5 | | | | | | | 56 |
| 10 | | | | | | | 49 |
| 30 | 99 | 97 | 90 | 77 | 89 | 77 | 34 |
| 60 | 96 | 96 | 86 | 67 | 86 | 67 | |
| 90 | 92 | 89 | 79 | 57 | 78 | 57 | |
| 120 | 86 | 87 | 72 | 56 | 74 | 54 | |

### <Example 16>

5 ml of a reaction solution, which was used in Example 15, consisting of 6.8% of mannose, 2.2% of fructose and 10.0% of ammonium carbonate was put into separate flasks of 100 ml capacity and subjected to condensation at temperatures of 40°C, 50°C, 60°C, 70°C and 80°C under a reduced pressure of 30 mmHg.

When water was evaporated, the same amount of distilled water as the evaporated amount was added and the above-described steps were repeated to execute a condensation treatment. At each step of the treatment (every 3 minutes to 5 minutes), a sample was collected from the condensed solution and the amount of the remaining ammonium carbonate was determined by a densimeter.

The results obtained are shown in Table 15.

From Table 15, it was clarified that by using this method, ammonium carbonate can be vaporized at a relatively low temperature in a short period of time in an effective manner, and complete removal from the reaction solution can be accomplished.

**Table 15**

| Treatment Temp. (°C) | Amount of remaining ammonium carbonate (%) | | |
|---|---|---|---|
| | 1^{st} Treatment | 2^{nd} Treatment | 3^{rd} Treatment |
| 40 | 34 | 16 | 4 |
| 50 | 40 | 16 | 6 |
| 60 | 19 | 4 | 0 |
| 70 | 4 | 0 | 0 |
| 80 | 4 | 0 | 0 |

### Industrial Applicability

As described above in detail, according to the method for producing mannose of the present invention, the yield of mannose, which had been about 25% in conventional methods using mannose isomerase, can be enhanced up to 90% or more.

Therefore, by using the method for producing mannose of the present invention, mannose or mannitol may be produced in large scale, inexpensively from fructose.

## Claims

1. A method for producing mannose comprising either:
(i) the isomerization of fructose, or a fructose-containing solution, a glucose isomerized sugar or an invert sugar; or
(ii) the reaction of glucose or a glucose-containing liquid with glucose isomerase and mannose isomerase whereby to produce mannose directly from said glucose or glucose-containing liquid
using mannose isomerase in the presence of a reducing sulfur-oxygen acid compound or an ammonium compound.

2. The method of claim 1 wherein said ammonium compound is selected from the group comprising ammonium chloride, ammonium sulfate, diammonium hydrogenphosphate ((NH₄)₂HPO₄), ammonium dihydrogenphosphate (NH₄H₂PO₄), ammonium hydrogencarbonate (NH₄HCO₃), ammonium carbonate ((NH₄)₂CO₃), hydroxylammonium (also known as hydroxylamine (NH₂OH)), hydroxylammonium chloride and hydroxylammonium sulfate.

3. The method of claim 1, wherein said sulfur-oxygen acid compound is selected from the group comprising sodium sulfite (Na₂SO₃), potassium sulfite (K₂SO₃), sodium metabisulfite (Na₂S₂O₅), (also know as sodium pyrosulfite or sodium disulfite), potassium metabisulfite (K₂S₂O₅) (also known as potassium pyrosulfite or potassium disulfite), sodium bisulfite (NaHSO₃), potassium bisulfite (KHSO₃), sodium hyposulfite (Na₂S₂O₄) and potassium hyposulfite (K₂S₂O₄).

4. A method as claimed in any one of claims 1 to 3 wherein said isomerization is performed in the presence of an ion-exchange resin treated with any one of the said reducing sulfur-oxygen acid compounds or said ammonium compounds.

5. A method as claimed in claim 4 wherein the reducing sulfur-oxygen acid compound or ammonium compound is a reducing sulfur-oxygen acid compound, and the ion-exchange resin is a strong basic ion-exchange resin or a mixed resin or a strong basic ion-exchange resin and a cation-exchange resin.

6. A method as claimed in claim 4 wherein the reducing sulfur-oxygen acid compound or ammonium compound is an ammonium compound and the ion-exchange resin is a cation-exchange resin or a mixed resin of a cation-exchange resin and an anion-exchange resin.

7. A method as claimed in either claim 5 or claim 6 wherein a reaction solution containing:
(i) fructose, a fructose-containing solution, a glucose isomerized sugar or an invert sugar; and
(ii) mannose isomerase is passed through a column packed with said compound-bonded resin, or said compound-bonded resin is immersed in said reaction solution.

8. A method as claimed in any preceding claim wherein said mannose is separated after said isomerization.

9. The method according to claim 8 wherein the separation is effected through removal of said reducing sulfur-oxygen acid compound or said ammonium compound by contacting the reaction solution resulting from said isomerization with an anion-exchange resin of a sulfurous acid type or a bisulfite type.

10. The method according to claim 6 wherein said ammonium compound is ammonium carbonate or ammonium bicarbonate and is removed from the reaction solution by vaporization, subsequent to said isomerization.

11. A method as claimed in any preceding claim wherein subsequent to said isomerization, the resultant reaction solution is used as a starting substrate for the production of mannitol.

## Patentansprüche

1. Ein Verfahren zum Erzeugen von Mannose, das entweder:
(i) die Isomerisierung von Fructose oder einer Fructose enthaltenden Lösung, eines glucoseisomerisierten Zuckers oder eines Invertzuckers; oder
(ii) die Reaktion von Glucose oder einer Glucose enthaltenden Flüssigkeit mit Glucoseisomerase und Mannoseisomerase umfasst, wobei zum Herstellen von Mannose direkt aus der Glucose oder der Glucose enthaltenden Flüssigkeit Mannoseisomerase in der Gegenwart einer reduzierenden Schwefel-Sauerstoff-Säure-Verbindung oder einer Ammoniumverbindung verwendet wird.

2. Verfahren nach Anspruch 1, worin die Ammoniumverbindung ausgewählt ist aus der Gruppe, die Ammoniumclorid, Ammoniumsulfat, Diammoniumhydrogenphosphat (NH₄)₂HPO₄), Ammoniumdihydrogenphosphat (NH₄H₂PO₄), Ammoniumhydrogencarbonat (NH₄HCO₃), Ammoniumcarbonat ((NH₄)₂CO₃), Hydroxylammonium (auch bekannt als Hydroxylamin (NH₂OH)), Hydroxylammoniumchlorid und Hydroxylammoniumsulfat enthält.

3. Verfahren nach Anspruch 1, worin die Schwefelsauerstoffsäureverbindung ausgewählt ist aus der Gruppe, die Natriumsulfit (Na₂SO₃), Kaliumsulfit (K₂SO₃), Natriummetabisulfit (Na₂S₂O₅) (auch bekannt als Natriumpyrosulfit oder Natriumdisulfit), Kaliummetabisulfit (K₂S₂O₅) (auch bekannt als Kaliumpyrosulfit oder Kaliumdisulfit), Natriumbisulfit (NaHSO₃), Kaliumbisulfit (KHSO₃), Natriumhyposulfit (Na₂S₂O₄) und Kaliumhyposulfit (K₂S₂O₄) enthält.

4. Verfahren nach einem der Ansprüche 1 bis 3, worin die Isomerisation in der Gegenwart von einem Ionenaustauschharz, das mit einem der reduzierenden Schwefalsauerstoffsäureverbindungen oder der Ammoniumverbindungen behandelt wurde, ausgeführt wird.

5. Verfahren nach Anspruch 4, worin die reduzierende Schwefelsauerstoffsäureverbindung oder Ammoniumverbindung eine reduzierende Schwefelsauerstoffsäureverbindung ist, und das Ionenaustauschharz ein stark basisches Ionenaustauschharz oder ein Mischharz oder ein stark basisches Ionenaustauschharz und ein Kationenaustauschharz ist.

6. Verfahren nach Anspruch 4, worin die reduzierende Schwefelsauerstoffsäureverbindung oder Ammoniumverbindung eine Ammoniumverbindung ist und das Ionenaustauschharz ein Kationenaustauschharz oder ein Mischharz aus einem Kationenaustauschharz und einem Anionenaustauschharz ist.

7. Verfahren entweder nach Anspruch 5 oder Anspruch 6, worin eine Reaktionslösung enthält:
(i) Fructose, eine Fructose enthaltende Lösung, einen glucoseisomerisierten Zucker oder einen Invertzucker, und
(ii) Mannoseisumerase wird durch eine Säule, die mit dem verbindungsgebund nen Harz befüllt ist, hindurchgeleitet, oder das verbindungsgebundene Harz wird in die Reaktionslösung eingetaucht.

8. Verfahren nach einem der vorhergehenden Ansprüche, worin die Mannose nach der Isomerisation separiert wird.

9. Verfahren nach Anspruch 8, worin die Separation durch Entfernen der reduzierenden Schwefelsauerstoffsäureverbindung oder der Ammoniumverbindung durch ein In-Kontaktbringen der Reaktionslösung, die aus der Isomerisation resultiert, mit einem Anionenaustauschharz vom Typ einer schwefligen Säure oder vom Typ eines Bisulfits ausgeführt wird.

10. Verfahren nach Anspruch 6, worin die Ammoniumverbindung Ammoniumcarbonat oder Ammoniumbicarbonat ist und von der Reaktionslösung durch Verdampfung im Anschluss an die Isomerisation entfernt wird.

11. Verfahren nach einem der vorhergehenden Ansprüche, worin im Anschluss an die Isomerisation die resultierende Reaktionslösung als Ausgangssubstrat für die Herstellung von Manitol verwendet wird.

## Revendications

1. Procédé de production de mannose comprenant :
(i) soit l'isomérisation du fructose, ou d'une solution contenant du fructose, d'un sucre de glucose isomérisé ou d'un sucre inverti ;
(ii) soit la réaction du glucose ou d'un liquide contenant du glucose avec une glucose isomérase et une mannose isomérase moyennant quoi du mannose est produit directement à partir dudit glucose ou dudit liquide contenant du glucose utilisant une mannose isomérase en présence d'un composé acide réducteur de soufre et d'oxygène ou d'un composé ammonium.

2. Procédé selon la revendication 1, dans lequel ledit composé ammonium est choisi dans le groupe comprenant le chlorure d'ammonium, le sulfate d'ammonium, l'hydrogénophosphate de diammonium ((NH₄)₂HPO₄), le dihydrogénophosphate d'ammonium (NH₄H₂PO₄), l'hydrogénocarbonate d'ammonium (NH₄HCO₃), le carbonate d'ammonium ((NH₄)₂CO₃), l'hydroxylammonium (également connu sous le nom d'hydroxylamine (NH₂OH)), le chlorure d'hydroxylammonium et le sulfate d'hydroxylammonium.

3. Procédé selon la revendication 1, dans lequel ledit composé acide de soufre et d'oxygène est choisi dans le groupe comprenant le sulfite de sodium (Na₂SO₃), le sulfite de potassium (K₂SO₃), le métabisulfite de sodium (Na₂S₂O₅), (également connu sous le nom de pyrosulfite de sodium ou disulfite de sodium), le métabisulfite de potassium (K₂S₂O₅) (également connu sous le nom de pyrosulfite de potassium ou disulfite de potassium), le bisulfite de sodium (NaHSO₃), le bisulfite de potassium (KHSO₃), l'hyposulfite de sodium (Na₂S₂O₄) et l'hyposulfite de potassium (K₂S₂O₄).

4. Procédé selon l'une quelconque des revendications 1 à 3 dans lequel ladite isomérisation est réalisée en présence d'une résine échangeuse d'ions traitée avec l'un quelconque desdits composés acide réducteur de soufre et d'oxygène ou desdits composés ammonium.

5. Procédé selon la revendication 4 dans lequel le composé acide réducteur de soufre et d'oxygène ou le composé ammonium est un composé acide réducteur de soufre et d'oxygène, et la résine échangeuse d'ions est une résine échangeuse d'ions fortement basique ou une résine mixte ou une résine échangeuse d'ions fortement basique et une résine échangeuse de cations.

6. Procédé selon la revendication 4 dans lequel le composé acide réducteur de soufre et d'oxygène ou le composé ammonium est un composé ammonium et la résine échangeuse d'ions est une résine échangeuse de cations ou une résine mixte composé d'une résine échangeuse de cation et d'une résine échangeuse d'anions.

7. Procédé selon la revendication 5 où la revendication 6 dans lequel une solution de réaction contenant :
(i) du fructose, une solution contenant du fructose, un sucre de glucose isomérisé ou un sucre inverti ; et ,
(ii) une mannose isomérase est passée à travers une colonne remplie avec ladite résine liée à un composé, ou ladite résine liée à un composé est immergée dans ladite solution de réaction.

8. Procédé selon l'une quelconque des revendications précédentes dans lequel ledit mannose est séparé après ladite isomérisation.

9. Procédé selon la revendication 8 dans lequel la séparation est réalisée en éliminant ledit composé acide réducteur de soufre et d'oxygène ou ledit composé ammonium en mettant en contact la solution de réaction résultant de ladite isomérisation avec une résine échangeuse d'anions de type acide sulfureux ou de type bisulfite.

10. Procédé selon la revendication 6 dans lequel ledit composé ammonium est un carbonate d'ammonium ou un bicarbonate d'ammonium et est éliminé de la solution de réaction par vaporisation, après ladite isomérisation.

11. Procédé selon l'une quelconque des revendications précédentes dans lequel après ladite isomérisation, la solution de réaction résultante est utilisée comme substrat de départ pour la production de mannitol.
